(19) Europäisches Patentamt / European Patent Office / Office européen des brevets

(11) **EP 3 265 198 B1**

(12) **EUROPEAN PATENT SPECIFICATION**

(45) Date of publication and mention
of the grant of the patent:
**26.04.2023   Bulletin 2023/17**

(21) Application number: **16706227.2**

(22) Date of filing: **25.02.2016**

(51) International Patent Classification (IPC):
**B01D 15/20** (2006.01)        **B01D 15/38** (2006.01)
**G01N 30/50** (2006.01)        **A61L 2/18** (2006.01)

(52) Cooperative Patent Classification (CPC):
**B01D 15/203; B01D 15/3804; G01N 30/50**

(86) International application number:
**PCT/EP2016/053996**

(87) International publication number:
**WO 2016/139128 (09.09.2016 Gazette 2016/36)**

(54) **SANITIZATION METHOD FOR AFFINITY CHROMATOGRAPHY MATRICES**

DESINFEKTIONSVERFAHREN FÜR AFFINITÄTSCHROMATOGRAPHIEMATRIZEN

PROCÉDÉ DE DÉSINFECTION POUR DES MATRICES DE CHROMATOGRAPHIE D'AFFINITÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **03.03.2015  GB 201503578**

(43) Date of publication of application:
**10.01.2018   Bulletin 2018/02**

(73) Proprietor: **Cytiva BioProcess R&D AB**
**751 84 Uppsala (SE)**

(72) Inventors:
 • **MONIE, Elin, Marianne**
**751 84 Uppsala (SE)**
 • **BJORKMAN, Tomas**
**751 84 Uppsala (SE)**
 • **GRONBERG, Anna**
**751 84 Uppsala (SE)**
 • **LJUNGLOF, Anders**
**751 84 Uppsala (SE)**
 • **RODRIGO, Gustav, Jose**
**751 84 Uppsala (SE)**
 • **TORSTENSON, Karin**
**751 84 Uppsala (SE)**
 • **WETTERHALL, Magnus, Carl, Erik**
**751 84 Uppsala (SE)**

(74) Representative: **Démoulin, Eva Lotta et al**
**Cytiva Sweden AB
Björkgatan 30
751 84 Uppsala (SE)**

(56) References cited:
**EP-B1- 1 224 462          WO-A1-2014/092636
WO-A1-2014/180852      WO-A1-2015/034566
US-A1- 2012 301 429      US-A1- 2013 344 567
US-B1- 6 248 683**

 • **ROGERS M ET AL: "Development of a rapid
sanitization solution for silicabased protein A
affinity adsorbents", JOURNAL OF
CHROMATOGRAPHY, ELSEVIER SCIENCE
PUBLISHERS B.V, NL, vol. 1216, no. 21, 22 May
2009 (2009-05-22), pages 4589-4596,
XP026460269, ISSN: 0021-9673, DOI:
10.1016/J.CHROMA.2009.03.065 [retrieved on
2009-03-28]**

Remarks:
The complete document including Reference
Table(s) and the Sequence Listing(s) can be
downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical field of the invention

The present invention relates to the field of affinity chromatography, and more specifically to a method of sanitizing affinity chromatography matrices.

Background of the invention

Immunoglobulins and immunoglobulin fragments represent the most prevalent biopharmaceutical products in either manufacture or development worldwide. The high commercial demand for and hence value of this particular therapeutic market has led to the emphasis being placed on pharmaceutical companies to maximize the productivity of their respective manufacturing processes whilst controlling the associated costs and maintaining product quality.

Affinity chromatography, typically on matrices comprising staphylococcal protein A or variants thereof, is normally used as one of the key steps in the purification of intact immunoglobulin molecules. The highly selective binding of protein A to the Fc chain of immunoglobulins provides for a generic step with very high clearance of impurities and contaminants. Similarly, matrices comprising *Peptostreptococcus* Protein L or variants thereof are useful for purifying e.g. Fabs or other fragments of antibodies like scFv, BiTEs, domain antibodies etc., which contain a kappa light chain.

Any bioprocess chromatography application requires comprehensive attention to definite removal of impurities and contaminants. Such impurities/contaminants can for example be non-eluted molecules adsorbed to the stationary phase or matrix in a chromatographic procedure, such as non-desired biomolecules or microorganisms, including for example proteins, carbohydrates, lipids, bacteria and viruses. The removal of such impurities/contaminants from the matrix is usually performed after a first elution of the desired product in order to regenerate the matrix before subsequent use. Such removal usually involves a procedure known as cleaning-in-place (CIP) and typically involves alkaline solutions.

Additionally, there is a need for regular sanitization of the matrix to inactivate any microorganisms or spores present in the column. This is typically carried out as a sanitization-in-place (SIP) procedure, either involving sodium hydroxide or acidic solutions, e.g. phosphoric/acetic acid mixtures containing bacteriostats such as benzyl alcohol. With matrices having proteinaceous ligands, it is however difficult to find conditions that effectively kill microorganisms and spores without damaging the ligands.

WO 2014/092636 A1 discloses a method for cleaning packed bed chromatography columns using primarily alkaline cleaning liquids. EP 1 224 462 B1 discloses a method comprising regeneration of a resin. US 2012/301429 A1 discloses i.a. a method for sanitizing a protein A affinity chromatography column. WO 2014/180852 A1 discloses a method for purifying antibodies. US 2013/344567 A1 discloses a method for immobilizing nucleic ligands. ROGERS M ET AL: "Development of a rapid sanitization solution for silicabased protein A affinity adsorbents" (JOURNAL OF CHROMATOGRAPHY, vol. 1216, no. 21, 22 May 2009 (2009-05-22), pages 4589-4596) discloses a Protein A chromatography media sanitization using acidified benzyl alcohol.

There is thus still a need in this field for efficient SIP methods that do not damage the ligands.

Summary of the invention

One aspect of the invention is to provide a sanitization and/or cleaning method for affinity chromatography media with proteinaceous ligands, which method does not substantially impair the function of the ligands. This is achieved with a method as defined in claim 1.

One advantage is that a high degree of bacteria and spore inactivation can be achieved. A further advantage is that product-related impurities such as tightly bound non-eluted antibodies or process-related impurities such as host cell proteins can be removed.

A second aspect of the invention is to provide a use of an oxidant solution for sanitization and/or cleaning of affinity chromatography media. This is achieved with a use as defined in the claims.

Further suitable embodiments of the invention are described in the dependent claims.

Definitions

The terms "antibody" and "immunoglobulin" are used interchangeably herein, and are understood to include also fragments of antibodies, fusion proteins comprising antibodies or antibody fragments and conjugates comprising antibodies or antibody fragments.

The terms a "kappa light chain-binding polypeptide" and "kappa light chain-binding protein" herein mean a polypeptide or protein respectively, capable of binding to the kappa light chain of an antibody and includes e.g. Protein L, and any variant, fragment or fusion protein thereof that has maintained said binding property.

The term "kappa light chain-containing protein" is used as a synonym of "immunoglobulin kappa light chain-containing protein" and herein means a protein comprising a kappa light chain derived from an antibody and includes any intact antibodies, antibody fragments, fusion proteins, conjugates or recombinant proteins containing a kappa light chain.

The term "oxidation-tolerant" herein means a material which retains at least 80%, such as at least 90%, of its functionality after 24 h incubation at 22 +/- 2 °C with an aqueous 0.1 M peracetic acid solution. The functionality can e.g. be the binding capacity for a target species such as an immunoglobulin, IgG, a monoclonal antibody or an antibody fragment.

The term "affinity chromatography matrix" herein means a separation matrix having ligands capable of binding a target species with an equilibrium dissociation constant $K_D$ less than about $10^{-5}$ M, such as between $10^{-14}$ and $10^{-6}$ M. $K_D$ is here defined as $K_D = [L]*[T]/[LT]$, where [L] is the concentration of free ligand, [T] is the concentration of free target species and [LT] is the concentration of ligand-target species complex. Most such ligands are proteins and can e.g. include bacterial immunoglobulin-binding proteins and their variants, antibodies, streptavidin, lectins etc. The target species can e.g. be a protein such as an immunoglobulin.

Brief description of figures

Fig. 1 shows chromatograms (UV detection) of recombinant Protein L before (a) and after (b) 1 h incubation in 0.1 M peracetic acid. x-axis time, y-axis UV absorbance (280 nm).

Fig. 2 shows chromatograms (UV detection) of single-chain camelid antibody against kappa chains (KappaSelect ligand) before (a) and after (b) 1 h incubation in 0.1 M peracetic acid. x-axis time, y-axis UV absorbance (280 nm).

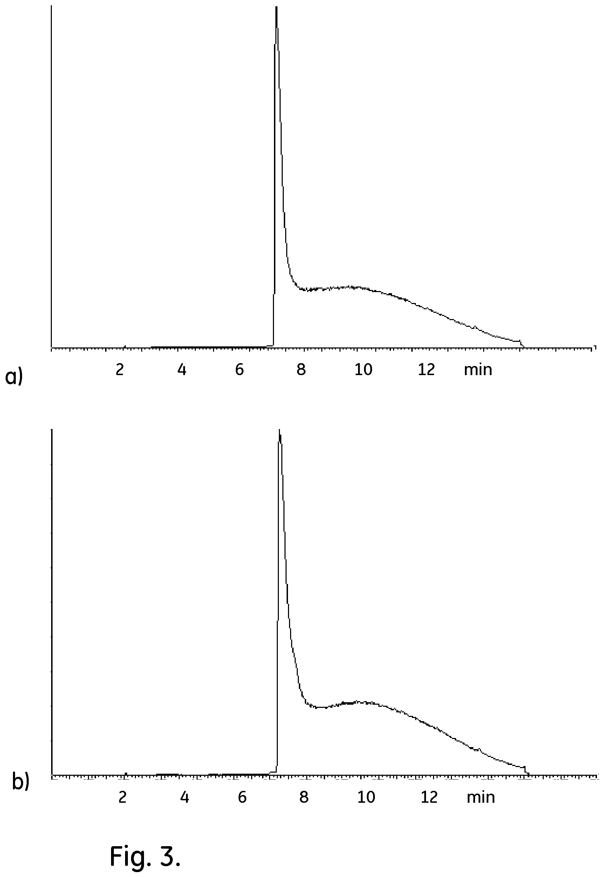

Fig. 3 shows chromatograms (MS detection) of recombinant Protein L before (a) and after (b) 1 h incubation in 0.1 M peracetic acid. x-axis time, y-axis MS response.

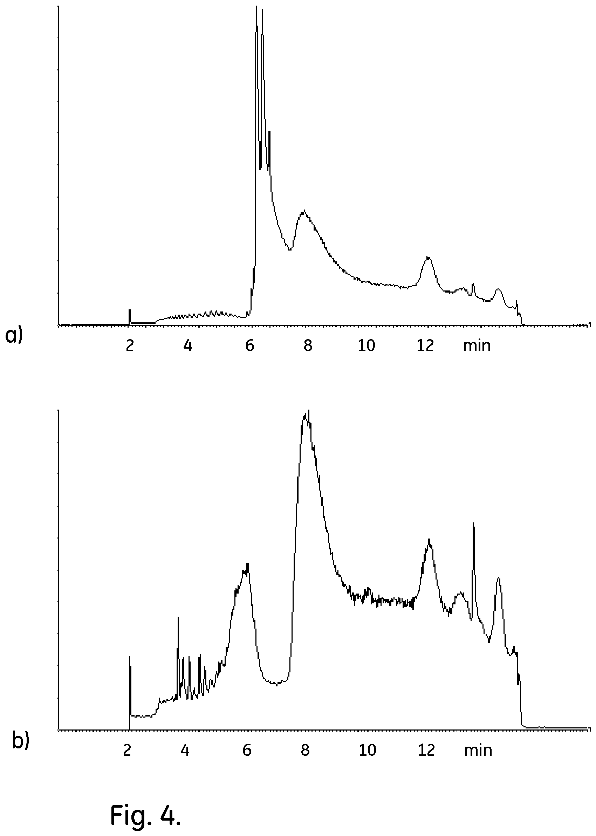

Fig. 4 shows chromatograms (MS detection) of single-chain camelid antibody against kappa chains (KappaSelect ligand) before (a) and after (b) 1 h incubation in 0.1 M peracetic acid. x-axis time, y-axis MS response.

Fig. 5 shows pressure-flow curves for a rigid agarose base matrix, a) untreated matrix and b) a sample of the same matrix after incubation with 30 mM peracetic acid solution.

Detailed description of embodiments

In one aspect the present invention discloses a method for cleaning or sanitization of an affinity chromatography matrix. This method comprises the steps of:

a) providing an affinity chromatography matrix having oxidation-tolerant proteinaceous ligands comprising *Staphylococcus* Protein A or an alkali-stabilized immunoglobulin-binding variant of *Staphylococcus* Protein A, coupled to a support. These proteins have a common single-chain structure characterized by the presence in the molecule of three different regions: the N-terminal region containing a signaling peptide responsible for the translocation of the protein across the cytoplasmic membrane and then detached; the functional region comprising several domains that determine the functional activity of the protein; and the C-terminal region denoted as a sorting signal responsible for anchoring of the protein in the cell wall. The functional regions are constructed on a single principle: they contain polypeptide repeats of one or several types. Highly homologous repeats of the same type can be organized as tandems. The number of repeats can vary and determine the protein heterogeneity in molecular weight and functional activity. Recombinant variants of these proteins to be used as affinity ligands often lack the C-terminal region, as it is not needed for this purpose. They can also have different N-terminal regions and selected point mutations in the domains to e.g. improve the alkali stability of the proteins.
b) contacting the matrix with a sanitization solution comprising at least one oxidant as defined by formula I,

$$R-O-O-H \qquad (I)$$

wherein R is hydrogen or an acyl group R'-C(O)-, with R' being a hydrogen or a methyl, ethyl or propyl group. Such oxidants include hydrogen peroxide $H_2O_2$, performic acid $HCO_3H$, peracetic acid $CH_3CO_3H$, perpropionic acid $CH_3CH_2CO_3H$ and perbutyric acid $CH_3CH_2CH_2CO_3H$. The pH of the sanitization solution can e.g. be 2-12, such as 2-4 or 2-3, which is beneficial for the sanitization effect and the concentration of the at least one oxidant can e.g. be 0.01-1.0 mol/l, such as 0.02-0.2 mol/l, 0.05-0.5 mol/l or 0.03-0.1 mol/l. The solution may contain a single oxidant or a mixture of oxidants according to formula I, e.g. a mixture of hydrogen peroxide with performic or peracetic acid. In the case of mixtures, the total concentration of oxidants defined by formula I can suitably be 0.01 - 1 mol/l, such as 0.02-0.2 mol/l, 0.05-0.5 mol/l or 0.03-0.1 mol/l. The solution may further comprise a carboxylic acid such as formic acid, acetic acid, propionic acid or butyric acid. If the solution comprises a peracid, the carboxylic acid can suitably be the corresponding carboxylic acid, i.e. a carboxylic acid R'-C(O)-OH having the same acyl group R'-C(O)-as the peracid R'C(O)OOH. The contacting may involve, or consist essentially of, incubating the matrix with the sanitization solution for e.g. 1 min - 24 h, such as 5 min-24 h or 10 min -24h. The incubation can suitably take place in a column packed with the matrix, or alternatively in a separate vessel with matrix from an unpacked column, and the incubation temperature can e.g. be room temperature or 15-30°C. The column can be a re-usable stainless steel column but it can also be a single use column, e.g. a column prepared from thermoplastic and elastomeric components. In the latter case, the thermoplastic and elastomeric materials can suitably be selected such that they are not significantly degraded by the oxidant solutions. Temperatures outside of the 15-30°C range can also be possible, particularly if some experimental work is carried out to find suitable concentrations and incubation times. As a first approximation it can be assumed that the usual Arrhenius temperature dependence can be applied, such that for a temperature increase of 10 °C, the incubation time may be decreased 2-3 times. The relationship between suitable concentrations and incubation times at constant temperature can be assumed to be approximately linear.

The immunoglobulin-binding domain(s) are derived from *Staphylococcus* Protein A. Within the disclosure, not covered by the claimed subject-matter, the immunoglobulin-binding domain(s) can also be derived from *Peptostreptococcus* Protein L or *Streptococcus* Protein G, such as from *Peptostreptococcus* Protein L. The immunoglobulin-binding domain(s) can e.g. have at least 80%, such as at least 90 or 95%, homology with Domain E, D, A, B or C of *Staphylococcus* Protein A, or with Protein Z (a variant of Domain B of Protein A) . In this context, the immunoglobulin-containing domain(s) can be defined by, or have at least 80%, such as at least 90 or 95% sequence homology with, an amino acid sequence selected from the group consisting of SEQ ID NO: 1-6. SEQ ID NO 7-11 are provided for reference.

SEQ ID NO:1- Domain E of Protein A
AQQ NAFYQVLNMP NLNADQRNGF IQSLKDDPSQ SANVLGEAQK LNDSQAPK

SEQ ID NO:2 - Domain D of Protein A

ADA QQNKFNKDQQ SAFYEILNMP NLNEEQRNGF IQSLKDDPSQ STNVLGEAKK LNESQAPK

SEQ ID NO:3 - Domain A of Protein A

A DNNFNKEQQ NAFYEILNMP NLNEEQRNGF IQSLKDDPSQ SANLLAEAKK LNESQAPK

SEQ ID NO:4 - Domain B of Protein A

ADNKFNKEQQ NAFYEILHLP NLNEEQRNGF IQSLKDDPSQ SANLLAEAKK LNDAQAPK

SEQ ID NO:5 - Domain C of Protein A

ADNKFNKEQQ NAFYEILHLP NLTEEQRNGF IQSLKDDPSV SKEILAEAKK LNDAQAPK

SEQ ID NO:6 - Protein Z

VDNKFNKEQQ NAFYEILHLP NLNEEQRNAF IQSLKDDPSQ SANLLAEAKK LNDAQAPK

SEQ ID NO:7 - Domain 1 of Protein L

SEEEVTIKAN LIFANGSTQT AEFKGTFEKA TSEAYAYADT LKKDNGEYTV DVADKGYTLN IKFAGKEKTPEE

SEQ ID NO:8 - Domain 2 of Protein L

PKEEVTIKAN LIYADGKTQT AEFKGTFEEA TAEAYRYADA LKKDNGEYTV DVADKGYTLN IKFAGKEKTPEE

SEQ ID NO:9 - Domain 3 of Protein L

PKEEVTIKAN LIYADGKTQT AEFKGTFEEA TAEAYRYADL LAKENGKYTV DVADKGYTLN IKFAGKEKTPEE

SEQ ID NO: 10 - Domain 4 of Protein L

PKEEVTIKAN LIYADGKTQT AEFKGTFAEA TAEAYRYADL LAKENGKYTA DLEDGGYTIN IRFAGKKVDEKPEE

SEQ ID NO: 11 - Domain 5 of Protein L

EKEQVTIKEN IYFEDGTVQT ATFKGTFAEA TAEAYRYADL LSKEHGKYTA

DLEDGGYTIN IRFAG

In some embodiments, the immunoglobulin-binding domain(s) can be derived from known variants of the native domains of SEQ ID NO: 1-6, such as the domains described in one or more of WO03080655A1, WO2008039141A1, EP1992692A1, EP2157099A1, EP2202310A2, EP2412809A1, EP2557157A1, EP2157099 and WO2013109302A2. The immunoglobulin-binding domain(s) can e.g. be defined by, or have at least 90%, such as at least 95 or 98% sequence homology with, an amino acid sequence selected from the group consisting of SEQ ID NO: 12-16.

SEQ ID NO:12 - Protein Z variant (WO03080655A1)

VDNKFNKEQQ NAFYEILHLP NLTEEQRNAF IQSLKDDPSQ SANLLAEAKK

LNDAQAPK

SEQ ID NO:13 - Protein Z variant (WO03080655A1)

VDAKFDKEQQ NAFYEILHLP NLTEEQRNAF IQSLKDDPSQ SANLLAEAKK LNDAQAPK

SEQ ID NO:14 - Domain C variant (WO2008039141A1)

ADNKFNKEQQ NAFYEILHLP NLTEEQRNAF IQSLKDDPSV SKEILAEAKK LNDAQAPK

SEQ ID NO:15 - Domain C variant (EP2557157A1)

ADNKFNKEQQ NAFYEILHLP NLTEEQRNAF IQELKDDPSV SKEILAEAKK LNDAQAPK

SEQ ID NO:16 - Domain C variant (WO3013109302A2)
FNKEQQ NAFYEILHLP NLTEEQRNGF IQSLKDDPSV SKEILAEAKK LNDAQAPK
SEQ ID NO: 17 - Protein A

AQQ NAFYQVLNMP NLNADQRNGF IQSLKDDPSQ SANVLGEAQK LNDSQAPK ADA QQNKFNKDQQ SAFYEILNMP NLNEEQRNGF IQSLKDDPSQ STNVLGEAKK LNESQAPK A DNNFNKEQQ NAFYEILNMP NLNEEQRNGF IQSLKDDPSQ SANLLAEAKK LNESQAPK ADNKFNKEQQ NAFYEILHLP NLNEEQRNGF IQSLKDDPSQ SANLLAEAKK LNDAQAPK ADNKFNKEQQ NAFYEILHLP NLTEEQRNGF IQSLKDDPSV SKEILAEAKK LNDAQAPK

SEQ ID NO:18 - Protein L (US5965390)

AVENKEETPETPETDSEEEVTIKANLIFANGSTQTAEFKGTFEKATSEAYAYADTLKKDN GEYTVDVADKGYTLNIKFAGKEKTPEEPKEEVTIKANLIYADGKTQTAEFKGTFEEATA EAYRYADALKKDNGEYTVDVADKGYTLNIKFAGKEKTPEEPKEEVTIKANLIYADGKT QTAEFKGTFEEATAEAYRYADLLAKENGKYTVDVADKGYTLNIKFAGKEKTPEEPKEE VTIKANLIYADGKTQTAEFKGTFAEATAEAYRYADLLAKENGKYTADLEDGGYTINIRF AGKKVDEKPEE

SEQ ID NO:19 - Protein G

AQHDEAVDAN SRGSVDASEL TPAVTTYKLV INGKTLKGET TTEAVDAATA EKVFKQYAND   NGVDGEWTYD DATKTFTVTE KPEVIDASEL TPAVTTYKLV INGKTLKGET TTKAVDAETA   EKAFKQYAND NGVDGVWTYD DATKTFTVTE MVTEVPLEST A

In certain embodiments of the method, the ligands comprise or consist essentially of homo- or heteromultimers of immunoglobulin-binding domains derived from a bacterial protein. This is to say that the ligands comprise a plurality of domains as discussed above. The domains in a ligand may all be the same (a homomultimer) or one or more of them can differ from the other(s) (a heteromultimer). In addition to the domains, the ligands may comprise linker structures between the domains, a leader or signal sequence at the N-terminus and a tail sequence at the C-terminus.

In some embodiments, the ligands comprise *Staphylococcus* Protein A or an alkali-stabilized immunoglobulin-binding variant of *Staphylococcus* Protein A. As stated above, Protein A comprises the five domains E, D, A, B, C in that order. Examples of commercially available matrices comprising *Staphylococcus* Protein A ligands include MabSelect™ and MabSelect Xtra (GE Healthcare), ProSep™-A and ProSep Ultra Plus (Merck-Millipore), Absolute™ (Novasep), CaptivA™ PriMab™ (Repligen) and Protein A Diamond (Bestchrom). Alkali-stabilized variants of Protein A are typically homo- or heteromultimers of modified Domain C or Protein Z units, as discussed e.g. in WO03080655A1, WO2008039141A1, EP1992692A1, EP2157099A1, EP2202310A2, EP2412809A1, EP2557157A1, EP2157099 and WO2013109302A2. Examples of commercially available matrices comprising alkali-stabilized immunoglobulin-binding variants of *Staphylococcus* Protein A include MabSelect SuRe and MabSelect SuRe LX (GE Healthcare), Eshmuno™ A (Merck-Millipore), Toyopearl™ AF-rProtein A (Tosoh Bioscience), Amsphere™ Protein A (JSR Life Sciences Inc.) and KanCapA™ (Kaneka Corp.).

According to the present disclosure and for reference only, ligands may comprise *Peptostreptococcus* Protein L or an alkali-stabilized immunoglobulin-binding variant of *Peptostreptococcus* Protein L. A commercially available matrix with Protein L ligands is Capto™ L (GE Healthcare). Alkali-stabilized variants of Protein L are discussed in co-pending applications PCT EP2015/079387 and PCT EP2015/079389. The ligands can also comprise an amino acid sequence defined by, or having at least 90%, such as at least 95 or 98% sequence homology with, an amino acid sequence selected from the group consisting of SEQ ID NO: 17-19.

In contrast to the bacterial protein-derived ligands discussed above, antibody-derived ligands have been found to be unstable towards the sanitization solutions used in the method of the invention. This is particularly the case for ligands derived from single-chain camelid antibodies, as described e.g. in WO0144301A1. Examples of matrices with such ligands include KappaSelect™, LambdaFabSelect™, VIIISelect™ and VIISelect™ (all GE Healthcare).

In some embodiments the method comprises, before step b), a step a') of contacting the matrix with a solution comprising an immunoglobulin to adsorb the immunoglobulin and subsequently contacting the matrix with an elution solution to desorb the immunoglobulin. Step a') can e.g. be repeated at least 10 times, such as at least 25 times before step b) is applied, but step b) may also be applied after each instance of step a'). Step a') may further include a cleaning-in-place step, e.g. using 10-500 or 10-100 mmol/l alkali, such as NaOH as a cleaning solution.

In certain embodiments, the content of viable bacteria, vegetative bacteria and/or spores is reduced by at least 3 $\log_{10}$, such as at least 5 $\log_{10}$ or at least 6 $\log_{10}$ in step b).

In some embodiments, the immunoglobulin- or IgG-binding capacity of the matrix after step b) is at least 95%, such as at least 97% of the immunoglobulin- or IgG-binding capacity of the matrix before step b).

The support (also called a base matrix) of the matrix can be of any suitable well-known kind, in particular an oxidation-tolerant support, such as a support whose pressure-flow performance is not changed by more than 20% after 24 h incubation at 22 +/- 2 °C with an aqueous 0.03 M or 0.1 M peracetic acid solution. A conventional affinity separation matrix is often of organic nature and based on polymers that expose a hydrophilic surface to the aqueous media used, i.e. expose hydroxy (-OH), carboxy (-COOH), carboxamido (-CONH$_2$, possibly in N- substituted forms), amino (-NH$_2$, possibly in substituted form), oligo- or polyethylenoxy groups on their external and, if present, also on internal surfaces. The solid support can suitably be porous. The porosity can be expressed as a Kav or Kd value (the fraction of the pore volume available to a probe molecule of a particular size) measured by inverse size exclusion chromatography, e.g. according to the methods described in Gel Filtration Principles and Methods, Pharmacia LKB Biotechnology 1991, pp 6-13. By definition, both Kd and Kav values always lie within the range 0-1. The Kav value can advantageously be 0.6 - 0.95, e.g. 0.7 - 0.90 or 0.6 - 0.8, as measured with dextran of Mw 110 kDa as a probe molecule. An advantage of this is that the support has a large fraction of pores able to accommodate both the proteinaceous ligands and immunoglobulins binding to the ligands and to provide mass transport of the immunoglobulins to and from the binding sites.

The proteinaceous ligands may be covalently coupled to the support, such as where they are attached to the support via conventional coupling techniques utilising e.g. thiol, amino and/or carboxy groups present in the ligand. Bisepoxides, epichlorohydrin, CNBr, N-hydroxysuccinimide (NHS) etc are well-known coupling reagents. Between the support and the ligands, a molecule known as a spacer can be introduced, which improves the availability of the ligand and facilitates the chemical coupling of the ligand to the support.

In some embodiments the matrix comprises 5 - 20, such as 5 - 15 mg/ml, 5 - 11 mg/ml or 8 - 11 mg/ml of the ligand coupled to the support. The amount of coupled ligand can be controlled by the concentration of ligand used in the coupling process, by the coupling conditions used and/or by the pore structure of the support used. As a general rule the absolute binding capacity of the matrix increases with the amount of coupled ligand, at least up to a point where the pores become significantly constricted by the coupled ligand. The relative binding capacity per mg coupled ligand will decrease at high coupling levels, resulting in a cost-benefit optimum within the ranges specified above.

In some embodiments the proteinaceous ligands are coupled to the support via multipoint attachment. This can suitably be done by using such coupling conditions that a plurality of reactive groups in the ligand react with reactive groups in the support. Typically, multipoint attachment can involve the reaction of several intrinsic reactive groups of amino acid residues in the sequence, such as amines in lysines, with the reactive groups on the support, such as epoxides, cyanate esters (e.g. from CNBr activation), succinimidyl esters (e.g. from NHS activation) etc. It is however also possible to deliberately introduce reactive groups at different positions in the ligands to affect the coupling characteristics. In order to provide multipoint coupling via lysines, the coupling reaction is suitably carried out at a pH where a significant fraction of the lysine primary amines are in the non-protonated nucleophilic state, e.g. at pH higher than 8.0, such as above 10.

In certain embodiments the ligands are coupled to the support via thioether bonds. Methods for performing such coupling are well-known in this field and easily performed by the skilled person in this field using standard techniques and equipment. Thioether bonds are flexible and stable and generally suited for use in affinity chromatography. In particular when the thioether bond is via a terminal or near-terminal cysteine residue on the ligand, the mobility of the coupled ligand is enhanced which provides improved binding capacity and binding kinetics. In some embodiments the ligand is coupled via a C-terminal cysteine provided on the protein as described above. This allows for efficient coupling of the cysteine thiol to electrophilic groups, e.g. epoxide groups, halohydrin groups etc. on a support, resulting in a thioether bridge coupling.

In certain embodiments the support comprises a polyhydroxy polymer, such as a polysaccharide. Examples of polysaccharides include e.g. dextran, starch, cellulose, pullulan, agar, agarose etc. Polysaccharides are inherently hydrophilic with low degrees of nonspecific interactions, they provide a high content of reactive (activatable) hydroxyl groups and they are generally stable towards alkaline cleaning solutions used in bioprocessing.

In some embodiments the support comprises agar or agarose. The supports used in the present invention can easily be prepared according to standard methods, such as inverse suspension gelation (S Hjertén: Biochim Biophys Acta 79(2), 393-398 (1964). Alternatively, the base matrices are commercially available products, such as crosslinked agarose beads sold under the name of SEPHAROSE™ FF (GE Healthcare). In an embodiment, which is especially advantageous for the sanitization method, the support is a rigid crosslinked agarose. Such agarose supports are highly crosslinked, e.g. according to the methods described in US6602990, US7396467 or US8309709. A rigid crosslinked agarose support in the form of spherical beads and packed to 20 cm bed height in a 2.6 cm inner diameter column gives a water flow velocity v (cm/h) at 3 bar back pressure which is at least $0.14 \times d^2$, where d is the volume-weighted median diameter (d50,v) of the beads in micrometers. The rigid crosslinked agarose is remarkably stable towards the oxidants used in the methods of the invention.

In certain embodiments the support, such as a polysaccharide or agarose support, is crosslinked, such as with hydroxyalkyl ether crosslinks. Crosslinker reagents producing such crosslinks can be e.g. epihalohydrins like epichlorohydrin, diepoxides like butanediol diglycidyl ether, allylating reagents like allyl halides or allyl glycidyl ether. Crosslinking is beneficial for the rigidity of the support and improves the chemical stability. Hydroxyalkyl ether crosslinks are alkali stable and do not cause significant nonspecific adsorption.

Alternatively, the solid support is based on synthetic polymers, such as polyvinyl alcohol, polyhydroxyalkyl acrylates, polyhydroxyalkyl methacrylates, polyacrylamides, polymethacrylamides etc. In case of hydrophobic polymers, such as matrices based on divinyl and monovinyl-substituted benzenes, the surface of the matrix is often hydrophilised to expose hydrophilic groups as defined above to a surrounding aqueous liquid. Such polymers are easily produced according to standard methods, see e.g. "Styrene based polymer supports developed by suspension polymerization" (R Arshady: Chimica e L'Industria 70(9), 70-75 (1988)). Alternatively, a commercially available product, such as SOURCE™ (GE Healthcare) is used. In another alternative, the solid support according to the invention comprises a support of inorganic nature, e.g. silica, glass, zirconium oxide etc.

In yet another embodiment, the solid support is in another form such as a surface, a chip, capillaries, or a filter (e.g. a membrane or a depth filter matrix).

As regards the shape of the matrix according to the invention, in one embodiment the matrix is in the form of a porous membrane. In an alternative embodiment, the matrix is in beaded or particle form that can suitably be porous. Matrices in beaded or particle form can be used as a packed bed or in a suspended form. Suspended forms include those known as expanded beds and pure suspensions, in which the particles or beads are free to move.

The present disclosure also provides for the use of a solution comprising an oxidant defined by formula I,

$$\text{R-O-O-H} \qquad (I)$$

wherein R is hydrogen or an acyl group R'-C(O)-, with R' being a hydrogen or a methyl, ethyl or propyl group, for sanitization of an affinity chromatography matrix having proteinaceous ligands coupled to a support. The proteinaceous ligands comprise or consist essentially of one or more immunoglobulin-binding domains derived from a bacterial protein as discussed above. The use may comprise the methods of any of the embodiments discussed above.

Further, the present disclosure provides for a method for sanitization of a chromatography matrix, comprising the steps of:

a) providing a chromatography matrix having oxidation-tolerant ligands coupled to a rigid crosslinked agarose support,
b) contacting the matrix with a sanitization solution comprising an oxidant defined by formula I,

$$\text{R-O-O-H} \qquad (I)$$

wherein R is hydrogen or an acyl group R'-C(O)-, with R' being a hydrogen or a methyl, ethyl or propyl group. The ligands can be the oxidation-tolerant proteinaceous ligands as discussed above, but they can also be e.g. cation exchange ligands (e.g. ligands comprising sulfopropyl, sulfoethyl or carboxymethyl groups), anion exchange ligands, (e.g. comprising trimethylammonium or diethylaminoethyl groups), hydrophobic ligands (e.g. comprising butyl, hexyl, octyl or phenyl groups) or multimodal groups (e.g. comprising cation or anion exchange groups in combination with hydrophobic groups). The ligands may be homogeneously distributed over the matrix or they may be exclusively or primarily located in one region of the matrix, e.g. the cores or shells of bead-shaped matrices.

As discussed above, the concentration of said oxidant in said sanitization solution can be e.g. 0.01 - 1 mol/l and the pH can be e.g. 2-12, such as 2-4 or 2-3. The matrix can in step b) be incubated with the sanitization solution for 1 min - 24 h, such as 5 min-24 h or 15 min - 3 h, and the matrix can e.g. be in the form of spherical beads having a median diameter (d50,v) of 10-200 micrometers, such as 30-100 micrometers.

Examples (all performed at room temperature = 22 +/- 2 °C)

Example 1 (Capto L sanitization study preference)

The purpose of this investigation was to evaluate the bactericidal and sporicidal effect on bacterial spores and bacteria of eight disinfectants as well as PBS as reference added to Capto L 50% slurry. The effect was tested with contact times of 0 and 15 minutes as well as 1, 4, and 24 hours.

The test was performed in slurries of Capto L 50% and disinfectants. Microorganisms were added at a concentration of approximately $10^7$ cfu/mL to the suspension and the reducing effect was evaluated after given time intervals by neutralizing the disinfectant. The efficacy of the neutralizer and its ability to recover the inoculated microorganisms was demonstrated by validating the method in parallel with the challenge test. The results were evaluated with regard to log10 reductions of the microorganisms *Bacillus subtilis* (spores) and *Pseudomonas aeruginosa* (vegetative bacteria).

$10^7$ cfu/mL of the microorganism was added to 10 mL of Capto L 50% slurry with disinfectant. Samples were mixed and duplicate samples of 0.1 mL were added to 50 mL of neutralizing agent (0.075 M phosphate buffer with 0.3% lecithin, 3.0 % Tween 80, 0.5% sodium thiosulfate and 0.1% L-histidine). The total solution was analyzed by filtrating fractions corresponding to 10-times serial dilutions. Filters were rinsed three times with 100 mL of 0.9 % NaCl. As positive control each microorganism was added to 50 mL of neutralizing agent, the same amount added as for the disinfectant challenge tests. The total solution was analyzed by filtrating fractions corresponding to 10-times serial dilutions. Filters were rinsed three times with 100 mL of 0.9% NaCl. Filters were incubated on TSA (Trypticase Soy Agar) at 30-35 °C for 2-5 days. Preliminary reading was performed after 1-3 days. The reduction of microorganisms was calculated as the $\log_{10}$ of the surviving microorganisms as compared to that of the positive control. The results show that among the tested disinfectants only 0.1 M peracetic acid and 0.6 % formic acid with 3 % hydrogen peroxide are capable of efficiently killing *B. subtilis* spores.

Table 1. Results ($\log_{10}$ reductions) of viable count of *Bacillus subtilis* when added to Capto L 50 % slurry containing various disinfectants after contact times of 0 and 15 minutes as well as 1, 4 and 24 hours. Results are mean values of duplicate samples from the slurries.

| Disinfectant | Log10 reduction of *Bacillus subtilis* spores | | | | |
|---|---|---|---|---|---|
| | 0 min | 15 min | 1 hour | 4 hours | 24 hours |
| PBS: 20 mM Phosphate, 150 mM NaCl, pH 7.4 (ref) | 0.0 | 0.0 | 0.0 | 0.0 | 0.0 |
| 0.5 M Acetic acid, 20% Ethanol | 0.0 | 0.0 | 0.0 | 0.0 | 0.0 |
| 100 mM NaOH, 60 % Ethanol | 0.0 | 0.3 | 0.5 | 1.1 | >5.3 |
| 8 M Urea, 0.5 M Acetic acid, 1 M NaCl, pH 2.5 | 0.8 | 0.7 | 1.0 | 0.8 | 1.0 |
| 8 M Urea. 0.5 M Acetic acid. pH 2.5 | 0.9 | 1.0 | 1.0 | 0.9 | 1.0 |
| 6 M Guanidine-HCl, 0.5 M Acetic acid | 0.8 | 0.8 | 0.8 | 1.0 | 1.2 |
| 0.1 M Peracetic acid | 5.6 | >6.9 | >6.9 | >6.9 | >6.9 |
| 0.6 % Formic acid, 3 % Hydrogen peroxide | 0.4 | >6.9 | >6.9 | >6.9 | >6.9 |
| 2 % Chlorhexidine digluconale | 1.3 | 1.2 | 0.7 | 1.6 | 0.2 |

Table 2. Results (logic reductions) of viable count of *Pseudomonas aeruginosa* when added to Capto L 50 % slurry containing various disinfectants after contact times of 0 and 15 minutes as well as 1, 4 and 24 hours. Results are mean values of duplicate samples from the slurries.

| Disinfectant | Log10 reduction of *Pseudomonas aeruginosa* | | | | |
|---|---|---|---|---|---|
| | 0 min | 15 min | 1 hour | 4 hours | 24 hours |
| PBS: 20 mM Phosphate, 150 mM NaCl, pH 7.4 (ref) | 0.3 | 0.3 | 0.3 | 0.3 | 0.4 |
| 0.5 M Acetic acid, 20% Ethanol | 6.9 | >6.9 | >6.9 | >6.9 | >6.9 |
| 100 mM NaOH, 60 % Ethanol | 6.9 | >6.9 | >6.9 | >6.9 | >6.9 |
| 8 M Urea, 0.5 M Acetic acid, 1 M NaCl, pH 2.5 | >6.9 | >6.9 | >6.9 | >6.9 | >6.9 |
| 8 M Urea. 0.5 M Acetic acid. pH 2.5 | >6.9 | >6.9 | >6.9 | >6.9 | >6.9 |
| 6 M Guanidine-HCl, 0.5 M Acetic acid | >6.9 | >6.9 | >6.9 | >6.9 | >6.9 |
| 0.1 M Peracetic acid | >6.9 | >6.9 | >6.9 | >6.9 | >6.9 |
| 0.6 % Formic acid. 3 % Hydrogen peroxide | >6.9 | >6.9 | >6.9 | >6.9 | >6.9 |
| 2 % Chlorhexidine digluconale | >6.9 | >6.9 | >6.9 | >6.9 | >6.9 |

Example 2 (ligand incubation, Biacore test)

The aim with this activity was to study the binding kinetics of different chromatography media ligands as a measure of the retained binding capability before and after sanitization with 0.1 M Peracetic acid (PAA). The kinetics was measured by Surface Plasmon Resonance on a Biacore™ instrument. The ligands tested were monomers (Z1) and tetramers (Z4) of SEQ ID NO: 13, recombinant Protein A (rPrA), recombinant Protein L (rPrL) and single-chain camelid antibodies against IgG kappa chains (KappaSelect ligand). Z1, Z4 and rPrA were tested with a monoclonal antibody immobilized on a Biacore chip, while rPrL and the KappaSelect ligand were tested with an immobilized Fab antibody fragment.

1. 1 ml 10 mg/ml Z1, Z4 and rPrA were reduced, alkylated and buffer exchanged to 10 mM NaCl and analyzed on a mass spectrometer (MS) to verify the reduction and alkylation.
2. 1 ml rPrL and KappaSelect ligand were diluted to 10 mg/ml.
3. Reference sample (0 h) was withdrawn, 100 µl and buffer exchanged to 50 mM Ammonium Bicarbonate.
4. 10 % 1 M PAA was added and the incubations started.
5. Samples were withdrawn after 1, 2, 4, 8, 16 and 24 h, 100 µl and buffer exchanged to 50 mM Ammonium Bicarbonate to stop the oxidation reaction.
6. The concentrations were measured in all samples by measuring the UV absorbance at 276 nm.
7. The samples were then analyzed on LC-MS and Biacore.

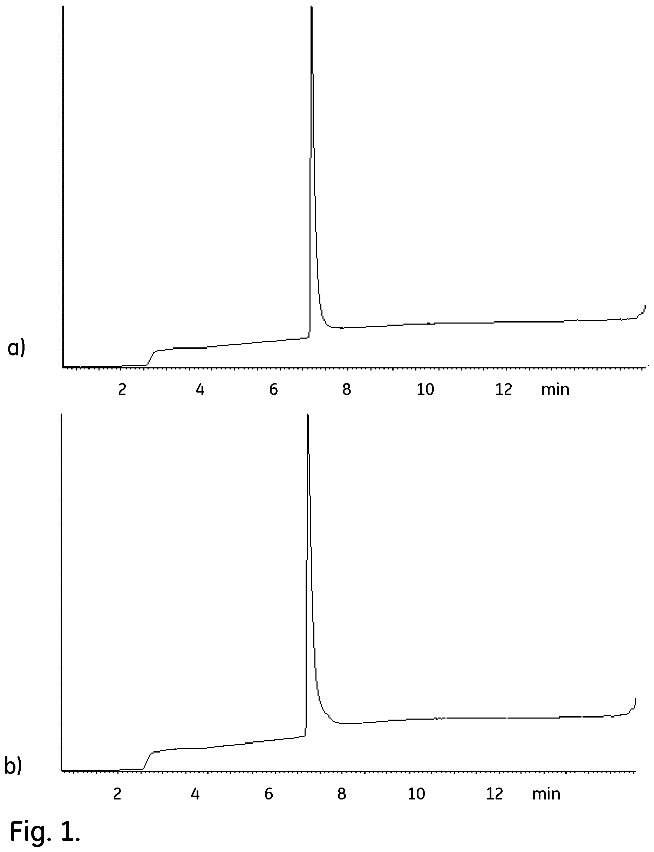

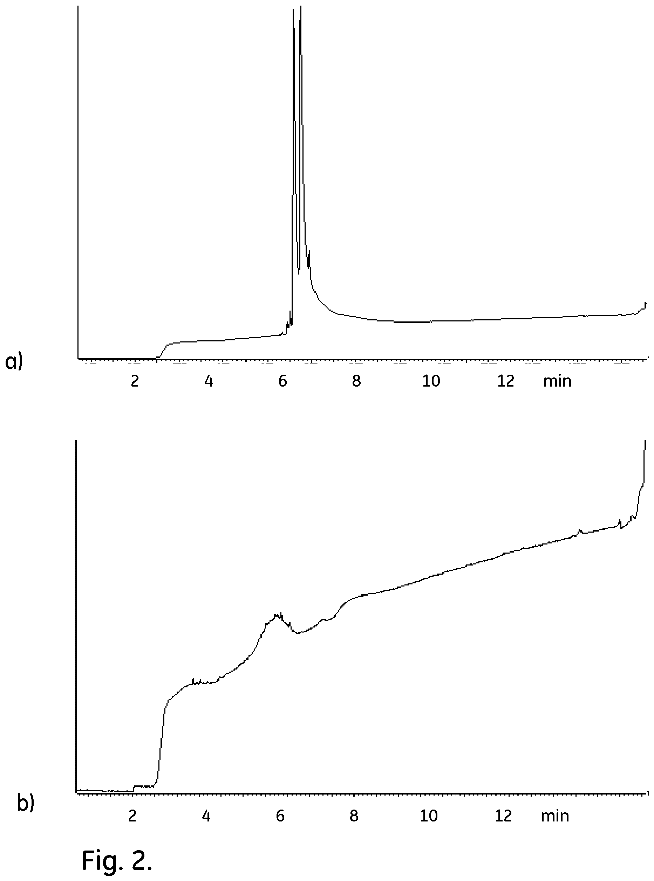

The Z1 ligand incubated for 0 to 16 h in 0.1 M PAA has similar affinity before and after SIP. The ligand after 24 h incubation does not change in off-rate but changes in on-rate, which also gives a shift in affinity.
The Z4 ligand incubated for 0 to 24 h in 0.1 M PAA has similar affinity before and after SIP. The rPrA ligand incubated for 0 to 24 h in 0.1 M PAA gives some loss in affinity with increasing SIP treatment. The ligand changes in both on-rate and off-rate, and gives a shift in affinity
The rPrL ligand incubated for 0 to 24 h in 0.1 M PAA has similar affinity before and after SIP. The The LC chromatograms before and after SIP (Fig 1 a and b) show no signs of degradation.
The KappaSelect ligand was so heavily degraded already after 1 h in 0.1 M PAA that it was not possible to perform any kinetics studies on it. The LC chromatograms before and after SIP (Fig 2 a and b) also show an essentially complete degradation.

Example 3 (matrix incubation)

The aim with these experiments was to test the Sanitization in place (SIP) compatibility of different affinity chromatography media. This was done by calculating the 10% breakthrough dynamic binding capacity (DBC) after SIP with 0.1 M Peracetic acid on six different chromatography media packed in PreDictor RoboColumn 600μl by using a TECAN robot. The initial DBC was measured on six corresponding reference columns which had not been subjected to SIP. The media were: Capto L (recombinant Protein L), KappaSelect (single-chain camelid antibodies against IgG kappa chains), MabSelect (recombinant Protein A), MabSelect SuRe (multimer of Protein Z variant with substituted asparagines), MabSelect SuRe LX (multimer of Protein Z variant with substituted asparagines) and MabSelect Xtra (recombinant Protein A). The support in all these media is rigid crosslinked agarose.

Methods

1. Wash/removal of storage solution: 10 column volumes (CV) (6× 1000 μl) 20 mM phosphate, 150 mM NaCl pH 7.4

2. Addition of 20 CV (12× 1000 μl) SIP solution (0.1 M peracetic acid)

3. Sealing of bottom and top of the mini-columns with column plugs, Parafilm and aluminium foil.

4. Incubation in SIP solution for 24 hours at room temperature

5. After 24h SIP incubation, wash: 10 CV (6× 1000 μl) 20 mM phosphate, 150 mM NaCl pH 7.4

6. Sample loading: 12*600μl Fab/Mab at different concentrations, residence time 6 min

7. Collection of unbound sample in UV-readable plates and measurement of UV absorbance at 280 (and 300 nm)

For DBC of columns not subjected to SIP, points 1, 6 and 7 were done.

Table 3. Dynamic binding capacities before and after SIP (24 h incubation in 0.1 M peracetic acid)

| Matrix | DBC control, no SIP (g/L) | DBC after SIP (g/L) | ΔDBC (g/L) |
|---|---|---|---|
| Capto L | 30 | 29 | -1 |
| KappaSelect | 20 | 0 | -20 |
| MabSelect | 52 | 51 | -1 |
| MabSelect SuRe | 50 | 51 | 1 |
| MabSelect SuRe LX | 63 | 63 | 0 |
| MabSelect Xtra | 59 | 60 | 1 |

Example 4 (matrix incubation Capto L)(reference)

A PreDictor™ (GE Healthcare) 96-well filter plate with 6 microliters Capto L in each well was subjected to the following procedure:

Removal of PreDictor storage solution (20 % Ethanol)
Wash: 3× 200 μl 20 mM phosphate, 150 mM NaCl pH 7.4
Wash 3×200 μl MilliQ
Addition of 600 μl SIP solutions and 20 mM phosphate, 150 mM NaCl pH 7.4 (control) (48 solutions and duplicates in plate)
Sealing of bottom and top of the plates with Parafilm
Incubation in SIP solutions for 4 or 24 hours with shaking at 450 rpm
Liquids were removed by centrifugation (500xg, 1 min).

The static binding capacity (SBC) of a Fab was determined by:

1. Removal of SIP solutions
2. Wash: 1×600 μl MilliQ, 1×600 μl PBS

3. Equilibration: 3×200 µl 20 mM phosphate, 150 mM NaCl pH 7.4

4. Sample loading: 200 µl Fab at a concentration of 2.5 mg/mL, incubation 10 min and 90 min at 1100 rpm

5. Collection of unbound sample in UV-readable plates and measurement of UV absorbance at 280 (and 254 nm)

Liquids were removed by centrifugation (500xg, 1 min).

The UV absorbance of the PBS buffer at 280 nm (path check on) was subtracted from the UV absorbance of the flow through fraction (A280-blank280)

The concentration in the flow through fraction was calculated using a standard curve (A280-blank280)/1.2796 Calculation of the SBC:

$$SBC = \frac{Vsample}{Vmedium}(Csample - Cunbound) - \frac{Vr*}{Vmedium} Cunbound$$

Vsample= 200µl
Vmedium=6 µl

$$Vr = 6 + 0.6* \ Vmedium = 9.6$$

Vr: The retained volume i.e. filter volume (6 µl) and liquid volume in resin (0.6*6)

Table 4 Remaining static capacities of Capto L for a Fab after 4 or 24h incubation in SIP solutions, compared to the control, 20 mM phosphate, 150 mM NaCl pH 7.4 (PBS). SBC values were determined both for 10 and 90 minutes incubation with the Fab sample.

| SIP solution | Relative remaining SBC (%) | | | |
|---|---|---|---|---|
| | 4h | | 24h | |
| | 10' | 90' | 10' | 90' |
| 100mM Peracetic acid | 109 | 101 | 107 | 105 |
| 500mM Peracetic acid | 106 | 103 | 97 | 102 |
| 5% H2O2 | 104 | 102 | 102 | 105 |
| 0.6% formic acid, 3% H2O2 | 108 | 105 | 104 | 106 |

Example 5 (matrix incubation with MS analysis)

200 mg wet matrix was washed with water and incubated 24 h with 0.1 M peracetic acid. The matrix was removed by filtration and the incubation solution was analyzed on a Waters AQUITY H-Class + Waters Xevo Q-TOFMS LC-MS system with a RPLC Cis RPC column. The presence of protein fragments in the incubation solution was taken as an indication of ligand degradation by the peracetic acid.

Seven matrices were tested (all from GE Healthcare), with different proteinaceous affinity ligands:

Kappa Select (single-chain camelid antibodies against IgG kappa chains)
LambdaFab Select (single-chain camelid antibodies against IgG lambda chains)
Factor VII Select (single-chain camelid antibodies against Factor VII)
Factor VIII Select (single-chain camelid antibodies against Factor VIII)
Capto L (recombinant Protein L)
MabSelect Xtra recombinant (Protein A)
MabSelect SuRe (multimer of Protein Z variant with substituted asparagines)

Table 5 Results from LC-MS analyses of 0.1 M peracetic acid incubation solutions.

| Matrix | Result |
|---|---|
| Kappa Select | Degradation of ligand |
| Lambda Fab Select | Degradation of ligand |
| Factor VII Select | Degradation of ligand |
| Factor VIII Select | Degradation of ligand |
| Capto L | No degradation, no ligand leakage |
| MabSelect Xtra | No degradation, small leakage of intact ligand |
| MabSelect SuRe | No degradation, small leakage of intact ligand |

Example 6 (Base matrix stability)

In order to see if the oxidant treatment had any negative properties of the agarose base matrix, a pressure-flow test of a rigid agarose base matrix was carried out on a non-treated matrix sample and a sample which had been incubated with oxidant.

Base matrix

The base matrix used was rigid cross-linked agarose beads of 88 micrometers (volume-weighted, d50V) median diameter, prepared according to the methods of US6602990 and with a pore size corresponding to an inverse gel filtration chromatography $K_D$ value of 0.70 for dextran of Mw 110 kDa, according to the methods described in Gel Filtration Principles and Methods, Pharmacia LKB Biotechnology 1991, pp 6-13.

Incubation

The base matrix was incubated in 30 mM aqueous peracetic acid for 24 h at room temperature and then washed with distilled water.

Column packing

300 ml sedimented gel was slurried with distilled water to give a slurry volume of 620 ml and packed in a FineLINE™ 35/600 column (GE Healthcare, Sweden) with inner diameter 35 mm. The packing pressure was 0.10 +/- 0.02 bar and the bed height was 300 +/- 10 mm.

Pressure-flow test

Distilled water was pumped through the column at stepwise increasing back pressures up to 7.5 bar. After each pressure increase step, the pump rate was kept constant for 5 min and the volumetric flow rate and back pressure were measured. Linear flow velocities (calculated as the volumetric flow rate divided by the inner cross section area of the column) were plotted against the back pressure and a maximum flow velocity was calculated from the curve, while the maximum pressure was taken as the back pressure at the maximum flow velocity.

Results

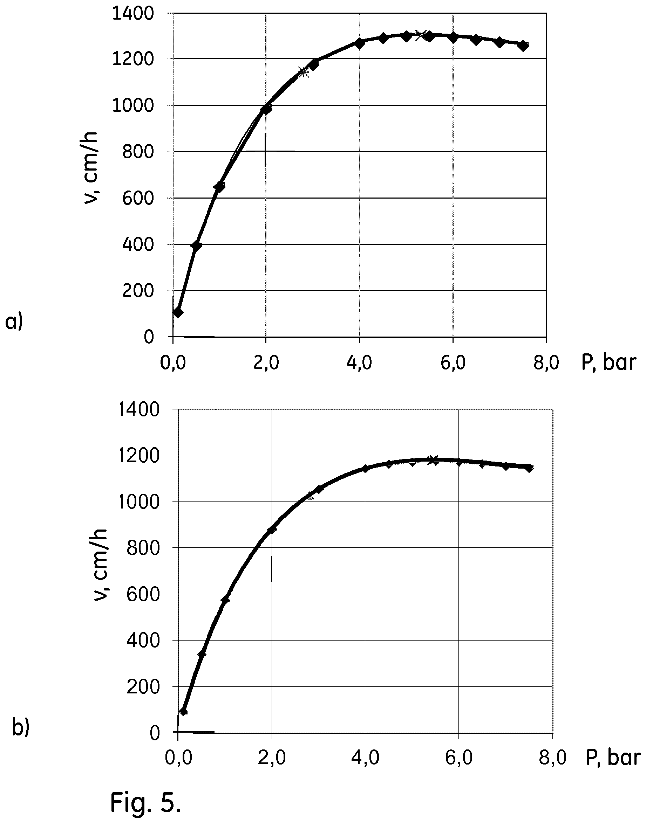

Fig 5 shows the flow velocity vs back pressure curves for a) non-incubated matrix and b) matrix after incubation. The maximum flow velocity was $1.30 \times 10^3$ cm/h before incubation and $1.18 \times 10^3$ cm/h after incubation, while the corresponding max pressures were 5.31 and 5.44 bar before and after incubation. The differences are within the experimental error limits of the method, indicating that the peracetic acid incubation did not affect the pressure-flow properties of the matrix. This was further corroborated by measuring the median diameter of the beads in a Coulter Counter, showing that the d50V median diameter was 88.3 micrometers before and 88.5 micrometers after incubation (no statistically significant difference). Also the pore size of the beads was unaffected as shown by the $K_D$ values for dextran of Mw 110 kDa of 0.696 before and 0.692 after incubation.

Example 7 (Cleaning, MabSelect SuRe)

Aliquots of MabSelect SuRe matrix were fouled with a monoclonal antibody (mAb) feed and then incubated with different cleaning solutions. After cleaning, the matrix was boiled in SDS-DTT buffer to release any proteins and the supernatant was run on an electrophoresis gel in an Amersham WB system (GE Healthcare, Sweden) to determine the amounts of residual foulant proteins.

Fouling

A 96-well filter plate with 20 microliters MabSelect SuRe in each well (PreDictor MabSelect Sure, GE Healthcare, Sweden) was equilibrated with 3*200 microliters PBS buffer per well and then loaded with 200 microliters mAb feed (CHO cell supernatant with 4 g/l mAb) per well and incubated for 30 min. After washing with 200 microliters PBS, the wells were eluted with 2*200 microliters 50 mM acetate buffer pH 3.5 The equilibration-loading-washing elution cycle was repeated for a total of five cycles and the wells were then equilibrated and distilled water added.

The liquid was removed by vacuum filtration or centrifugation after each step (sample load, wash, elution, cleaning)

Cleaning

The wells were washed with 3*200 microliters distilled water and then incubated with 300 microliters CIP solution 1 for 15 min. After removal of the CIP 1 solution, they were washed with 2*300 microliters PBS and 2*300 microliters water. They were then incubated with 300 microliters CIP solution 2 for 15 min and washed as above. The CIP 1 and CIP 2 solutions are listed in Table 6.

Table 6. CIP solutions and amounts of residual foulant proteins on MabSelect SuRe

| CIP solution 1 | CIP solution 2 | Residual foulant (scan intensity) |
|---|---|---|
| PBS (control) | PBS (control) | 32400 |
| PBS | 100 mM NaOH | 3810 |
| 30 mM peracetic acid | PBS | 4580 |
| 30 mM peracetic acid | 100 mM NaOH | 595 |
| 100 mM peracetic acid | PBS | 2030 |
| 100 mM peracetic acid | 100 mM NaOH | 347 |
| 100 mM DTT in Tris | 100 mM NaOH | 1150 |

Analysis

The matrix in the wells was prelabeled with Cy5 fluorescent dye using the Amersham WB Cy5 labeling kit, by adding 50 microliters labeling buffer + 5 microliters Cy5 and incubating 30 minutes. 50 microliters SDS-DTT buffer was then added and boiled for 5 min with the matrix. The supernatants were loaded on an Amersham WB Gel Card and run together with prelabeled mAb and MabSelect SuRe ligand references plus a set of molecular weight standards.The Gel Card was scanned and the scan signals integrated to give numbers corresponding to the amount of residual foulant retrieved from the matrix.

The results show that 30 mM peracetic acid is almost as effective as 100 mM NaOH, while 100 mM peracetic acid is more effective. Particularly good results were obtained with a serial combination of oxidant and alkali.

Example 8 (Cleaning, Capto L)(reference)

This was performed as Example 7, except that a 20 microliter PreDictor Capto L plate, with Protein L-functional Capto L matrix in the wells was used and that the foulant was a domain antibody (dAb) feed, consisting of supernatant from a heat-treated E. Coli culture, with periplasmic expression of the dAb.

Table 6. CIP solutions and amounts of residual foulant proteins on Capto L

| CIP solution 1 | CIP solution 2 | Residual foulant (scan intensity) |
| --- | --- | --- |
| PBS (control) | PBS (control) | 42700 |
| PBS | 15 mM NaOH | 6920 |
| 30 mM peracetic acid | PBS | 13600 |
| 30 mM peracetic acid | 15 mM NaOH | 1660 |
| 100 mM peracetic acid | PBS | 4150 |
| 100 mM peracetic acid | 15 mM NaOH | 1960 |
| 100 mM DTT in Tris | 15 mM NaOH | 7250 |

Also here, the peracetic acid has a considerable cleaning effect and the effect is further enhanced by serial combination with 15 mM alkali.

**Claims**

1. A method for cleaning or sanitization of an affinity chromatography matrix, comprising the steps of:

   a) providing an affinity chromatography matrix having oxidation-tolerant proteinaceous ligands comprising *Staphylococcus* Protein A or an alkali-stabilized immunoglobulin-binding variant of *Staphylococcus* Protein A, coupled to a support,
   b) contacting said matrix with a sanitization solution comprising at least one oxidant defined by formula I,

   $$R - O - O - H \qquad (I)$$

   wherein R is hydrogen or an acyl group R'-C(O)-, with R' being a hydrogen or a methyl, ethyl or propyl group.

2. The method of claim 1, wherein said proteinaceous ligands comprise or consist essentially of one or more immunoglobulin-binding domains derived from a bacterial protein.

3. The method of claim 1 or 2, wherein the concentration of said oxidant in said sanitization solution is 0.01 - 1 mol/l.

4. The method of any preceding claim, wherein the pH of said sanitization solution is 2-4 or 2-3.

5. The method of any preceding claim, wherein:

   i) said oxidant is selected from the group consisting of hydrogen peroxide, performic acid and peracetic acid;
   ii) said sanitization solution comprises a mixture of at least two oxidants defined by formula I, such as a mixture of hydrogen peroxide with performic or peracetic acid;
   iii) the total concentration of oxidants defined by formula I is 0.01 - 1 mol/l; and/or
   iv) in step b) the matrix is incubated with said sanitization solution for 1 min - 24 h, such as 5 min - 24 h or 15 min - 3 h.

6. The method of any one of claims 2-5, wherein said immunoglobulin-binding domains;

   i) have at least 80%, such as at least 90 or 95%, homology with Domain E, D, A, B or C of *Staphylococcus* Protein A, with Protein Z
   and/or
   ii) are defined by, or have at least 90%, such as at least 95 or 98% sequence homology with, an amino acid sequence selected from the group consisting of SEQ ID NO: 12-16.

7. The method of any preceding claim, wherein said support:

   i) comprises porous particles or a porous membrane;
   ii) is selected from the group consisting of silica, glass and hydroxyfunctional polymers;

iii) is a crosslinked polysaccharide; and/or
iv) is crosslinked agarose, such as rigid crosslinked agarose.

8. The method of any one of claims 2-7, comprising, before step b), a step a') of contacting said matrix with a solution comprising an immunoglobulin to adsorb said immunoglobulin and subsequently contacting said matrix with an elution solution to desorb said immunoglobulin, wherein optionally step a') is repeated at least 10 times, such as at least 25 times before step b).

9. The method of any preceding claim, wherein said matrix is packed in a chromatography column and wherein optionally said chromatography column is a single use column or a column manufactured from thermoplastic and elastomeric components.

**Patentansprüche**

1. Verfahren zur Reinigung oder Desinfektion einer Affinitätschromatographie-Matrix, umfassend die folgenden Schritte:

   a) Bereitstellen einer Affinitätschromatographie-Matrix mit oxidationstoleranten proteinartigen Liganden, die *Staphylococcus* Protein A oder eine alkalistabilisierte Immunglobulin-bindende Variante von *Staphylococcus* Protein A umfassen, gekoppelt an einen Träger,
   b) In-Kontakt-bringen der Matrix mit einer Desinfektionslösung, die mindestens ein durch die Formel I definiertes Oxidationsmittel umfasst,

   $$R - O - O - H \qquad (I)$$

   wobei R für Wasserstoff oder eine Acylgruppe R'-C(O)- steht, wobei R' für Wasserstoff oder eine Methyl-, Ethyl- oder Propylgruppe steht.

2. Verfahren nach Anspruch 1, wobei die proteinartigen Liganden eine oder mehrere von einem bakteriellen Protein abgeleitete Immunglobulin-bindende Domänen umfassen oder im Wesentlichen aus diesen bestehen.

3. Verfahren nach Anspruch 1 oder 2, wobei die Konzentration des Oxidationsmittels in der Desinfektionslösung 0,01 - 1 mol/l beträgt.

4. Verfahren nach einem vorstehenden Anspruch, wobei der pH-Wert der Desinfektionslösung 2-4 oder 2-3 beträgt.

5. Verfahren nach einem vorstehenden Anspruch, wobei:

   i) das Oxidationsmittel ausgewählt ist aus der Gruppe, bestehend aus Wasserstoffperoxid, Perameisensäure und Peressigsäure;
   ii) die Desinfektionslösung ein Gemisch aus mindestens zwei durch die Formel I definierten Oxidationsmitteln umfasst, wie ein Gemisch aus Wasserstoffperoxid mit Perameisensäure oder Peressigsäure;
   iii) die Gesamtkonzentration der durch Formel I definierten Oxidationsmittel 0,01 - 1 mol/l beträgt; und/oder
   iv) in Schritt b) die Matrix mit der Desinfektionslösung für 1 Min. - 24 Std., wie für 5 Min. - 24 Std. oder 15 Min. - 3 Std., inkubiert wird.

6. Verfahren nach einem der Ansprüche 2-5, wobei die Immunglobulin-bindenden Domänen;

   i) mindestens 80%, wie mindestens 90 oder 95%, Homologie mit der Domäne E, D, A, B oder C von *Staphylococcus* Protein A, mit Protein Z aufweisen und/oder
   ii) durch eine Aminosäuresequenz definiert sind oder mindestens 90%, wie mindestens 95 oder 98% Sequenzhomologie mit einer Aminosäuresequenz aufweisen, die ausgewählt ist aus der Gruppe, bestehend aus SEQ ID NO: 12-16.

7. Verfahren nach einem vorstehenden Anspruch, wobei der Träger:

   i) poröse Partikel oder eine poröse Membran umfasst;

ii) ausgewählt aus der Gruppe, bestehend aus Kieselsäure, Glas und hydroxyfunktionellen Polymeren;
iii) ein vernetztes Polysaccharid ist; und/oder
iv) vernetzte Agarose, wie rigide vernetzte Agarose ist.

8. Verfahren nach einem der Ansprüche 2-7, umfassend vor Schritt b) einen Schritt a') des In-Kontakt-bringens

der Matrix mit einer Lösung, die ein Immunglobulin umfasst, um das Immunglobulin zu adsorbieren und des anschließenden In-Kontakt-Bringens der Matrix mit einer Elutionslösung, um das Immunglobulin zu desorbieren, wobei optional Schritt a') mindestens 10-mal, wie mindestens 25-mal vor Schritt b), wiederholt wird.

9. Verfahren nach einem vorstehenden Anspruch, wobei die Matrix in eine Chromatographiesäule gepackt wird und wobei optional die Chromatographiesäule eine Säule zur einmaligen Verwendung oder eine aus thermoplastischen und elastomeren Komponenten hergestellte Säule ist.

**Revendications**

1. Procédé de nettoyage ou de désinfection d'une matrice de chromatographie d'affinité, comprenant les étapes de :

a) fourniture d'une matrice de chromatographie d'affinité présentant des ligands protéiques tolérants à l'oxydation comprenant la protéine A du *staphylocoque* ou un variant de liaison d'immunoglobuline aux alcalins stabilisés de la protéine A du *staphylocoque,* couplée à un support,
b) mise en contact de ladite matrice avec une solution de désinfection comprenant au moins un oxydant défini par la formule I,

$$R - O - O - H \qquad (1)$$

dans lequel R est de l'hydrogène ou un groupe acyle R'-C(O)-, avec R' étant un hydrogène ou un groupe méthyle, éthyle ou propyle.

2. Procédé selon la revendication 1, dans lequel lesdits ligands protéiques comprennent ou se composent essentiellement d'un ou plusieurs domaines de liaison d'immunoglobuline dérivés d'une protéine bactérienne.

3. Procédé selon la revendication 1 ou 2, dans lequel la concentration dudit oxydant dans ladite solution de désinfection est de 0,01 à 1 mole/l.

4. Procédé selon une quelconque revendication précédente, dans lequel le pH de ladite solution de désinfection est de 2 à 4 ou 2 à 3.

5. Procédé selon une quelconque revendication précédente, dans lequel :

i) ledit oxydant est sélectionné à partir du groupe constitué de peroxyde d'hydrogène, acide performique et acide péracétique.
ii) ladite solution de désinfection comprend un mélange d'au moins deux oxydants définis par la formule I, tel qu'un mélange de péroxyde d'hydrogène avec de l'acide performique ou péracétique ;
iii) la concentration totale d'oxydants définis par la formule I est de 0,01 à 1 mole/l ; et/ou
iv) à l'étape b) la matrice est incubée avec ladite solution de désinfection pendant 1 minute à 24 heures, telle que 5 minutes à 24 heures ou 15 minutes à 3 heures.

6. Procédé selon l'une quelconque des revendications 2 à 5, dans lequel lesdits domaines de liaison d'immunoglobuline ;

i) présentent au moins 80 %, tel qu'au moins 90 ou 95 %, d'homologie avec le domaine E, D, A, B ou C de la protéine A du *staphylocoque,* avec la protéine Z et/ou
ii) sont définis par, ou présentent au moins 90 %, tels qu'au moins 95 ou 98 % d'homologie de séquence avec, une séquence d'acides aminés sélectionnée à partir du groupe constitué de SEQ ID NO: 12-16.

7. Procédé selon une quelconque revendication précédente, dans lequel ledit support :

i) comprend des particules poreuses ou une membrane poreuse ;
ii) est sélectionné à partir du groupe constitué de silice, verre et polymères hydroxyfonctionnels ;
iii) est un polysaccharide réticulé ; et/ou
iv) est de l'agarose réticulé, tel qu'un agarose réticulé rigide.

8. Procédé selon l'une quelconque des revendications 2 à 7, comprenant avant l'étape b), une étape a') de mise en contact

de ladite matrice avec une solution comprenant une immunoglobuline pour adsorber ladite immunoglobuline et la mise en contact ultérieure de ladite matrice avec une solution d'élution pour désorber ladite immunoglobuline, dans lequel en option l'étape a') est répétée au moins 10 fois, tel qu'au moins 25 fois avant l'étape b).

9. Procédé selon une quelconque revendication précédente, dans lequel ladite matrice est emballée dans une colonne de chromatographie et dans lequel en option ladite colonne de chromatographie est une colonne à usage unique ou une colonne fabriquée à partir de composants thermoplastiques et élastomères.

a)

b)

Fig. 1.

Fig. 2.

a)

b)

Fig. 3.

a)

b)

Fig. 4.

a)

b)

Fig. 5.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014092636 A1
- EP 1224462 B1
- US 2012301429 A1
- WO 2014180852 A1
- US 2013344567 A1
- WO 03080655 A1
- WO 2008039141 A1
- EP 1992692 A1
- EP 2157099 A1
- EP 2202310 A2
- EP 2412809 A1
- EP 2557157 A1
- EP 2157099 A
- WO 2013109302 A2
- WO 3013109302 A2
- US 5965390 A
- EP 2015079387 W
- EP 2015079389 W
- WO 0144301 A1
- US 6602990 B
- US 7396467 B
- US 8309709 B

### Non-patent literature cited in the description

- **ROGERS M et al.** Development of a rapid sanitization solution for silicabased protein A affinity adsorbents. *JOURNAL OF CHROMATOGRAPHY,* 22 May 2009, vol. 1216 (21), 4589-4596
- Gel Filtration Principles and Methods. *Pharmacia LKB Biotechnology,* 1991, 6-13
- **S HJERTÉN.** *Biochim Biophys Acta,* 1964, vol. 79 (2), 393-398
- **R ARSHADY.** Styrene based polymer supports developed by suspension polymerization. *Chimica e L'Industria,* 1988, vol. 70 (9), 70-75